# EUROPÄISCHE PATENTSCHRIFT

(11) **EP 3 370 637 B1**
(45) Veröffentlichungstag und Bekanntmachung des Hinweises auf die Patenterteilung: **30.10.2019**
(21) Anmeldenummer: 16808557.9
(22) Anmeldetag: 01.11.2016
(51) Int. Cl.: A61C 7/00, A61C 7/10, A61C 9/00

(54) **VERFAHREN ZUR HERSTELLUNG EINES DISTRAKTORS ZUR SKELETTALEN BEFESTIGUNG AN EINEM KIEFER**
METHOD FOR PRODUCING A DISTRACTOR FOR SKELETAL FIXATION TO A JAW
PROCÉDÉ DE RÉALISATION D'UN DISTRACTEUR POUR FIXATION AU SQUELETTE D'UNE MÂCHOIRE

(30) Priorität: 03.11.2015 DE 102015118853
(43) Veröffentlichungstag der Anmeldung: 12.09.2018
(73) Patentinhaber: Technische Universität Dresden, 01069 Dresden (DE)
(72) Erfinder: BOTZENHART, Ute Ulrike, 01309 Dresden (DE); WEILAND, Bernhard, 01277 Dresden (DE); GEDRANGE, Tomasz, 01219 Dresden (DE)
(74) Vertreter: Riechelmann & Carlsohn Patentanwälte PartG mbB
(86) Internationale Anmeldenummer: PCT/DE2016/100514
(87) Internationale Veröffentlichungsnummer: WO 2017/076389

(56) Entgegenhaltungen:
- EP-A1- 2 687 168
- WO-A1-2008/031562
- US-A1- 2009 087 810
- ALKAN ET AL: "Mandibular symphyseal distraction osteogenesis: review of three techniques", INTERNATIONAL JOURNAL OF ORAL AND MAXILLOFACIAL SURGERY, COPENHAGEN, DK, Bd. 36, Nr. 2, 3. Februar 2007 (2007-02-03), Seiten 111-117, XP005866230, ISSN: 0901-5027, DOI: 10.1016/J.IJOM.2006.11.005 in der Anmeldung erwähnt

## Beschreibung

Die Erfindung betrifft ein Verfahren zur Herstellung eines Unterkieferdistraktors. Sie betrifft ferner einen Unterkieferdistraktor, der mittels des erfindungsgemäßen Verfahrens erhalten werden kann.

Die Unterkiefersymphyse besteht aus zwei paarigen Anteilen, die bereits sehr früh, d.h. im ersten Lebensjahr verknöchern (Becker MJ. Mandibular symphysis (medial suture) closure in modern Homo sapiens: preliminary evidence from archaeological populations. American journal of physical anthropology 1986; 69: 499-501). Damit ist die knöcherne Breite des Unterkiefers durch konventionelle kieferorthopädische Maßnahmen nur bedingt beeinflussbar. Bei ausgeprägtem Platzmangel stellt die Symphysendistraktion ein alternatives Verfahren zur Extraktionstherapie permanenter Zähne zwecks Platzbeschaffung dar. Die erstmals von Guerrero et al. 1997 beschriebene Methode ist im Vergleich zu anderen chirurgischen Verfahren ein noch relativ neues Verfahren (Guerrero CA, Bell WH, Contasti Gl et al.Mandibular widening by intraoral distraction osteogenesis. The British journal of oral & maxillofacial surgery 1997; 35: 383-392). Unter den bekannten Distraktoren unterscheidet man grundsätzlich zahngetragene, dentale und knochengetragene, skelettale Distraktoren. Dentale Distraktoren werden beispielsweise in DE 197 12 791 A1, DE 102 13 823 A1 und EP 1 088 520 A2, beschrieben. Skelettale Distraktoren werden beispielsweise in US 6,302,687 B2 und DE 601 04 297 T2 beschrieben.

Da bei dental getragenen Distraktoren unerwünschte Zahnbewegungen auftreten können (Seeberger R, Kater W, Davids R et al. Changes in the mandibular and dentoalveolar structures by the use of tooth borne mandibular symphyseal distraction devices. Journal of cranio-maxillo-facial surgery: official publication of the European Association for Cranio-Maxillo-Facial Surgery 2011; 39: 177-181), werden vermehrt skelettal getragene Distraktoren bevorzugt, die beidseits lateral der Unterkiefersymphyse fixiert werden und einen transgingivalen Anteil mit Aktivator-Schraube im Bereich des Vestibulums besitzen.

Die Form der Unterkiefersymphyse und des Unterkieferkorpus unterliegen einer hohen interindividuellen Variabilität (Jung SY, Shin SY, Lee KH et al. Analysis of mandibular structure using 3D facial computed tomography. Otolaryngology--head and neck surgery: official journal of American Academy of Otolaryngology-Head and Neck Surgery 2014; 151: 760-764). Das hat zur Folge, dass alle vorgefertigten Systeme intraoperativ angepasst, d. h. vom Chirurgen angebogen werden müssen. Dies führt zum einen zu einer erhöhten Ungenauigkeit der Passung und Unvorhersagbarkeit der anschließend wirkenden Kraftvektoren, die mit der Varianz der Platzierung und Passgenauigkeit korreliert sind (Basciftci FA, Korkmaz HH, Iseri H et al. Biomechanical evaluation of mandibular midline distraction osteogenesis by using the finite element method. American journal of orthodontics and dentofacial orthopedics: official publication of the American Association of Orthodontists, its constituent societies, and the American Board of Orthodontics 2004; 125: 706-715). Zum anderen weist der Übergangsbereich zwischen skelettaler Fixationsmöglichkeit und transgingivalem Anteil mit Schraube eine erhöhte Frakturanfälligkeit auf bzw. der epigingivale Anteil ist oft sehr voluminös und kann vor allem bei flacher Umschlagfalte zur Irritation der Weichgewebe und Entzündungen beitragen. Ferner ist bei vorgefertigten Systemen aufgrund der Anpassungsnotwendigkeit mit einer erhöhten Operationszeit zu rechnen.

Die Anpassung der vorgefertigten, konventionellen Systeme durch den Chirurgen an die individuellen Gegebenheiten eines jeden Patienten kann bereits präoperativ an einem Modell des Unterkiefers, beispielsweise einem Stereolithographiemodell oder einem 3D-Printmodell, erfolgen. Eine gewisse Passungenauigkeit muss aber immer intraoperativ ausgeglichen werden. Des Weiteren kann eine Materialschwächung durch Torsion und Biegung nicht vermieden werden. Für die oben aufgeführten Probleme gibt es derzeit also keine zufriedenstellenden Lösungen.

Den Nachteilen eines dental getragenen Distraktors stehen somit die Nachteile des skelettal getragenen Distraktors gegenüber. Mit dental getragenen Distraktoren sind unerwünschte Zahnbewegungen und Zahnkippungen aufgrund des Kraftangriffes (Seeberger et al, a.a.O.); eine Einengung des Zungenraumes; ein erhöhtes Kariesrisiko; ein erschwerter Zugang für den Chirurgen, da die Eingliederung präoperativ erfolgt; und die Gefahr der Lockerung, und zwar intraoperativ oder während der Konsolidierungsphase und eine schwierige Replatzierung (Alkan A, Ozer M, Bas B et al. Mandibular symphyseal distraction osteogenesis: review of three techniques. International journal of oral and maxillofacial surgery 2007; 36: 111-117) verbunden. Skelettal getragene Distraktoren bieten lediglich eine relative Passungenauigkeit; erfordern eine intraoperative Anpassung; es gibt eine Varianz bei der Positionierung; es besteht das Risiko von Zahnwurzelschädigungen; die Kraftvektoren sind relativ unvorhersehbar, insbesondere führt eine zu kaudale Platzierung zu einer geringen Öffnung des Distraktionsspaltes im Zahnbogen; es besteht eine Frakturanfälligkeit (Uckan S, Veziroglu F, Arman A. Unexpected breakage of mandibular midline distraction device: case report. Oral surgery, oral medicine, oral pathology, oral radiology, and endodontics 2006; 102: e21-25); eine Irritation von Weichgeweben durch voluminösen Anteil im Vestibulum kann auftreten; die Operationszeit kann aufgrund der Anpassungsnotwendigkeit verlängert sein; und schließlich ist eine Exposition des Foramen mentale zur Lagefindung notwendig, was die Gefahr einer Schädigung des Nervus mentalis und seiner Seitenäste nach sich ziehen kann (Apostolakis D, Brown JE. The dimensions of the mandibular incisive canal and its spatial relationship to various anatomical landmarks of the mandible: a study using cone beam computed tomography. The International journal of oral & maxillofacial implants 2013; 28: 117-124; Miller RJ, Edwards WC, Boudet C et al. Maxillofacial anatomy: the mandibular symphysis. The Journal of oral implantology 2011; 37: 745-753; Pires CA, Bissada NF, Becker JJ et al. Mandibular incisive canal: cone beam computed tomography. Clinical implant dentistry and related research 2012; 14: 67-73).

"XP5866230, Mandibular symphyseal distraction osteogenesis: review of three techniques von A. Alkan" beschreibt einen wichtigen Stand der Technik für die Erfindung.

Aufgabe der Erfindung ist es, die Nachteile nach dem Stand der Technik zu beseitigen. Es soll insbesondere ein Verfahren zur Herstellung eines Kieferdistraktors angegeben werden, mit dem eine bessere Passungenauigkeit des Kieferdistraktors erreicht, der Zeitaufwand zum Anpassen des Unterkieferdistraktors an den Unterkiefer eines Patienten verringert und die Varianz bei der Platzierung des Unterkieferdistraktors eliminiert werden kann. Ferner soll ein Kieferdistraktor angegeben werden, der mittels des erfindungsgemäßen Verfahrens hergestellt werden kann.

Diese Aufgabe wird durch die Merkmale der Ansprüche 1 und 12 gelöst. Zweckmäßige Ausgestaltungen der Erfindungen ergeben sich aus den Merkmalen der Unteransprüche.

Nach Maßgabe der Erfindung ist ein Verfahren zur Herstellung eines Distraktors zur skelettalen Befestigung an einem Unterkiefer eines Patienten vorgesehen, das die Schritte umfasst:
(a) Anfertigen einer dreidimensionalen Aufnahme des Kiefers des Patienten oder von Teilen des Kiefers;
(b) Anfertigen eines virtuellen Modelles eines patientenspezifischen Distraktors unter Verwendung der dreidimensionalen Aufnahme; und
(c) Herstellung des Distraktors oder Teilen davon anhand des virtuellen Modelles mittels 3D-Druckens.

Das erfindungsgemäße Verfahren ist zur Herstellung eines Distraktors zur skelettalen Befestigung am Unterkiefer, insbesondere am vorderen Unterkiefer des Patienten geeignet.

Es ist grundsätzlich geboten, die Belastung des Patienten mit Strahlung so gering wie möglich zu halten. Es kann daher ausreichend sein, eine dreidimensionale Aufnahme nur eines Teiles des Kiefers zu fertigen. Das gilt insbesondere dann, wenn eine dreidimensionale Aufnahme nur eines Teiles des Kiefers ausreichend ist, um den patientenspezifischen Distraktor herzustellen. Zur sprachlichen Vereinfachung wird die Erfindung nachstehend anhand einer dreidimensionalen Aufnahme des Kiefers erläutert. Die Erfindung kann jedoch gleichermaßen mit einer dreidimensionalen Aufnahme nur eines Teils des Kiefers ausgeführt werden.

Die dreidimensionale Aufnahme kann mittels eines Röntgenverfahrens oder anderen 3D-Aufnahmetechniken angefertigt werden. Beispielsweise kann die dreidimensionale Aufnahme mittels bildgebender Verfahren wie Computertomographie, Magnetresonanztomographie oder dentaler Volumentomographie angefertigt werden. Typischerweise werden bei diesen Verfahren zunächst mehrere zweidimensionale Aufnahmen erhalten. Diese Aufnahmen können mittels einer Datenverarbeitungsvorrichtung, beispielsweise einem Computer, zu einer dreidimensionalen Aufnahme zusammengesetzt werden. Die dreidimensionale Aufnahme kann als Volumengrafik, beispielsweise einer aus Voxeln zusammengesetzten Volumengrafik, bereitgestellt werden. Aus einer solchen Volumengrafik können wiederum dreidimensionale Ansichten des Kiefers erzeugt werden. Der Begriff "dreidimensionale Aufnahme", wie er in der vorliegenden Erfindung verwendet wird, bezeichnet sowohl die unmittelbar mittels der bildgebenden Verfahren erhaltenen Aufnahmen als auch die daraus berechneten Darstellungen des Kiefers, vorausgesetzt, dass die Daten eine dreidimensionale Darstellung des Kiefers zulassen. Eine dreidimensionale Aufnahme kann eine 3D-Darstellung des Kiefers ermöglichen.

Die dreidimensionale Aufnahme kann als medizinischer Datensatz bereitgestellt werden. Ein solcher Datensatz wird auch als medizinischer 3D-Datensatz bezeichnet. Der medizinische Datensatz enthält zweckmäßigerweise nur noch die Daten, die zur Bildung eines virtuellen Modells des Distraktors in Schritt (b) des erfindungsgemäßen Verfahrens erforderlich sind. Der medizinische Datensatz kann die Grundlage für ein virtuelles Modell des Kiefers sein. Es ist jedoch auch möglich, alternativ oder zusätzlich die dreidimensionale Aufnahme unmittelbar, d. h. ohne vorherige Bildung eines medizinischen Datensatzes, zur Bildung des virtuellen Modells des Distraktors einzusetzen.

Unabhängig davon, ob ein medizinischer Datensatz oder unmittelbar eine dreidimensionale Aufnahme des Kiefers eingesetzt werden, um ein virtuelles Modell des patientenspezifischen Distraktors zu erzeugen, kann vorgesehen sein, dass zunächst ein virtuelles Modell des Kiefers gebildet wird. Das virtuelle Modell des Kiefers des Patienten kann dann dazu genutzt werden, das virtuelle Modell des patientenspezifischen Distraktors zu bilden. Das virtuelle Modell des Kiefers kann unter Anwendung mathematischer Verfahren aus dem medizinischen Datensatz oder der dreidimensionalen Aufnahme gebildet werden. Zur Ausführung eines solchen mathematischen Verfahrens kann eine Datenverarbeitungsvorrichtung, beispielsweise ein Computer, eingesetzt werden. Das virtuelle Modell kann als 3D-CAD-Datensatz bereitgestellt werden.

Es kann vorgesehen sein, dass die dreidimensionale Aufnahme, der daraus erhaltene medizinische Datensatz oder das virtuelle Modell des Kiefers genutzt werden, um Referenzstrukturen des Kiefers zu bestimmen. Referenzstrukturen sind beispielsweise anatomische Merkmale des Kiefers, die patientenspezifisch sind. Derartige Referenzstrukturen sind beispielsweise die Lage der Foramina mentale, der Verlauf der Nervenkanäle sowie dentale Strukturen. Die Lage der Referenzstrukturen kann genutzt werden, um das virtuelle Modell des Distraktors an die patientenspezifischen Besonderheiten des Kiefers anzupassen. Mittels der Referenzstrukturen kann beispielsweise sichergestellt werden, dass Verankerungsstellen zur Befestigung des patientenspezifischen Distraktors am Kiefer des Patienten nicht in Bereichen des Kiefers vorgenommen werden müssen, die aus medizinischer Sicht ungeeignet oder ungünstig sind. Solche Bereiche sind zum Beispiel Bereiche, in denen Nervenkanäle verlaufen oder enden, beispielsweise die Foramina mentale. Damit können Schädigungen der Nerven bei der Anbringung des Distraktors verhindert werden. Umgekehrt können bestimmte Referenzstrukturen genutzt werden, um Bereiche des Kiefers zu bestimmen, die aus medizinischer Sicht besonders geeignet sind, Verankerungsstellen des Distraktors aufzunehmen. Die Referenzstrukturen können genutzt werden, um ein virtuelles Modell des patientenspezifischen Distraktors anzufertigen, der an die anatomischen Besonderheiten des Patienten exakt angepasst ist.

Das virtuelle Modell des Distraktors lässt eine Anpassung an die patientenspezifischen Besonderheiten zu. Es vermindert daher den Zeitaufwand, der mit einer interoperativen Anpassung herkömmlicher Distraktoren verbunden ist. Es ist zur Anfertigung des erfindungsgemäßen Distraktors möglich, Referenzmerkmale und/oder andere anatomische Merkmale, beispielsweise Nervenkanäle oder die Foramina mentale in der dreidimensionalen Aufnahme zu veranschaulichen. Das gilt ebenso für den aus der dreidimensionalen Aufnahme erhaltenen medizinischen Datensatz oder das virtuelle Modell des Kiefers. Insbesondere ermöglicht es das erfindungsgemäße Verfahren, die Verankerungsstellen zur Befestigung des patientenspezifischen Distraktors patientenspezifisch zu bestimmen.

Erfindungsgemäß ist in Schritt (c) vorgesehen, dass der patientenspezifische Distraktor oder Teile davon anhand des virtuellen Modelles hergestellt werden. Dabei wird eine Verfahrensweise eingesetzt, die eine schnelle Fertigung des Distraktors ermöglicht. Diese Verfahrensweise ist das 3D-Drucken, stärker bevorzugt das 3D-Metalldrucken und besonders bevorzugt das 3D-Titandrucken. Bei 3D-Titandruckern wird Titan als Werkstoff eingesetzt. Es ist nicht erforderlich, den gesamten Distraktor anhand des virtuellen Modells herzustellen, um eine vollständige Anpassung des Distraktors an die patientenspezifischen Merkmale des Kiefers, insbesondere seine anatomischen Besonderheiten zu erreichen. Es ist jedoch bevorzugt, den gesamten patientenspezifischen Distraktor anhand des virtuellen Modelles herzustellen.

Nicht alle Teile des Distraktors bedürfen zwingend einer patientenspezifischen Anpassung. Das gilt insbesondere für die Teile des Distraktors, die nicht direkt am Kiefer des Patienten befestigt werden. Teile des Distraktors, die direkt am Kiefer des Patienten befestigt werden, bilden den skelettalen Anteil des Distraktors. Teile des Distraktors, die über andere Teile des Distraktors am Kiefer befestigt sind, bilden den nicht-skelettalen Anteil des Distraktors. Es kann vorgesehen sein, dass nur der skelettale Anteil des Distraktors anhand des virtuellen Modelles hergestellt wird, während der nicht-skelettale Anteil aus vorgefertigten Elementen besteht. Beispielsweise kann der Distraktionszylinder, mit dem der Abstand zwischen den beiden Fixierungsarmen verändert werden kann, ein Standard-, d. h. vorgefertigtes Element sein. Hingegen müssen die Fixierungsarme, die an dem Kiefer befestigt werden, vollständig angepasst werden. Daher kann es ausreichend sein, nur die Fixierungsarme anhand des virtuellen Modells herzustellen und für die anderen Teile des Distraktors Standardelemente zu verwenden. Es kann auch vorgesehen sein, die Fixierungsarme und die Verbindungselemente, die zum Befestigen der Fixierungsarme am Kiefer erforderlich sind, anhand des virtuellen Modells herzustellen und für die anderen Teile des Distraktors Standardelemente zu verwenden. Bei den Verbindungselementen kann es sich um Schrauben oder erste Steckverbindungselemente handeln, die in Bohrungen, die im Kiefer an den Verankerungsstellen geschaffen werden, eingeschraubt oder eingesteckt werden können. Der skelettale Anteil des Distraktors umfasst somit die Fixierungsarme. Er kann ferner auch die Verbindungselemente umfassen. Der nicht-skelettale Anteil des Distraktors umfasst den Distraktionszylinder. Er kann - wenn die Verbindungselemente nicht dem skelettalen Anteil zugeordnet sind - die Verbindungselemente umfassen. Er kann ferner alle weiteren Teile des Distraktors, beispielsweise Führungsstifte, umfassen. Die Zuordnung der Verbindungselemente zum skelettalen Anteil oder zum nicht-skelettalen Anteil kann ein Arzt, insbesondere ein Zahnarzt treffen.

Es können zweite Steckverbindungen vorgesehen sein, wenn ein vorgefertigtes Bauteil, z. B. eine vorgefertigte Schraube, mit einem patientenspezifischen Teil des Distraktors verbunden wird. Die zweite Steckverbindung ist dann nicht zwischen Distraktor und Kiefer, sondern zwischen dem vorgefertigtem und dem patientenspezifischen Bauteil angeordnet.

Das erfindungsgemäße Verfahren kann mittels einer Vorrichtung ausgeführt werden, die eine Einheit zur Aufnahme des dreidimensionalen Bildes; eine Datenverarbeitungseinheit zur Erzeugung des virtuellen Modells des patientenspezifischen Distraktors, sowie eine Einheit zum Herstellen des patientenspezifischen Distraktors oder Teilen davon aufweist. Bei der Einheit zur Aufnahme des dreidimensionalen Bildes kann es sich um eine Röntgeneinrichtung, beispielsweise einen dentalen Volumentomografen oder einen Computertomografen handeln. Bei der Datenverarbeitungseinheit zur Erzeugung des virtuellen Modells des patientenspezifischen Distraktors kann es sich um einen Computer handeln. Die Datenverarbeitungseinheit kann eine Software oder ein Softwaremodul aufweisen und ausführen, mit der das virtuelle Modell des patientenspezifischen Distraktors generiert werden kann. Bei der Einheit zum Herstellen des patientenspezifischen Distraktors kann es sich um einen 3D-Drucker handeln. Die Vorrichtung kann Kommunikationsmittel, beispielsweise Kabel, aufweisen, um Daten zwischen den Einheiten zu transportieren. Die Datenverarbeitungseinheit kann eine Software oder ein Softwaremodul aufweisen und ausführen, die zur Steuerung der Einheit zur Aufnahme des dreidimensionalen Bildes, der Einheit zum Herstellen des patientenspezifischen Distraktors oder beiden Einheiten geeignet ist. Die Datenverarbeitungseinheit kann ferner eine Software oder ein Softwaremodul aufweisen und ausführen, mit der ein virtuelles Modell des Kiefers oder eines Teils davon erzeugt wird. Sie kann ferner eine Software oder ein Softwaremodul aufweisen und ausführen, mit der aus der dreidimensionalen Aufnahme ein medizinischer Datensatz erzeugt wird. Das erfindungsgemäße Verfahren zur Herstellung eines Distraktors kann ein rein digital gesteuertes Verfahren sein.

Nach Maßgabe der Erfindung ist ferner ein Distraktor zur skelettalen Befestigung an einem Kiefer oder einem anderen Gesichtsschädelknochen eines Patienten vorgesehen. Der Distraktor wird mittels des erfindungsgemäßen Verfahrens hergestellt, so dass er an den Kiefer oder anderen Gesichtsschädelknochen des Patienten exakt angepasst ist. Der erfindungsgemäße Distraktor ist somit ein patientenspezifischer Distraktor, der exakt an die anatomischen Gegebenheiten des Patienten angepasst ist. Das erfindungsgemäße Verfahren erlaubt die Herstellung eines Distraktors, der die anatomische Variabilität der Merkmale von Patientenkiefern berücksichtigt. Damit kann ein Distraktor erhalten werden, der nur geringen Zeitaufwand zur Anbringung an den Kiefer oder anderen Gesichtsschädelknochen des Patienten benötigt. Aufgrund der exakten Anpassung des erfindungsgemäßen Distraktors an die anatomischen Merkmale des Patienten verbessern sich darüberhinaus die Heilungschancen des Patienten. Insbesondere sinkt die Komplikationsrate. Mit unerwarteten Nebenwirkungen, vergleichbar mit konventionellen Distraktoren, ist nicht zu rechnen.

Das erfindungsgemäße Verfahren und der erfindungsgemäße Distraktor ermöglichen somit eine exakte und eindeutige intraoperative Platzierung des Distraktors am Kiefer. Damit verkürzt sich die Operationszeit und das Operationsrisiko sinkt. Der chirurgische Zugang kann entsprechend minimalinvasiv gewählt werden. Eine Exposition des Nervus mentalis ist vermeidbar. Das Risiko von Zahnwurzelschädigungen ist reduziert. Die Kraftvektoren können präoperativ bestimmt werden, beispielsweise anhand der dreidimensionalen Aufnahme oder des virtuellen Modells des Disktraktors. Das erfindungsgemäße Verfahren und der erfindungsgemäße Distraktor ermöglichen eine Vollindividualisierung des Distraktors mit exakter interaoperativer Adaptation an die Symphysenregion des Kiefers, insbesondere des Unterkiefers. Das erfindungsgemäße Verfahren ermöglicht die Herstellung eines Distraktors, der neben den anatomischen Strukturen auch die biomechanischen Komponenten und die chirurgischen Komponenten, insbesondere die Schnittführung, berücksichtigt. Der erfindungsgemäße Disktraktor kann am Kieferknochen platziert werden. Er wird nicht in den Kieferknochen implantiert. Der erfindungsgemäße Disktraktor ist zur temporären Anwendung bestimmt. Er ist insbesondere zur transversalen Erweiterung des Unterkiefers geeignet.

Die Platzierung des erfindungsgemäßen Distraktors ist ohne Hilfsmittel möglich. Der erfindungsgemäße Distraktor kann aufgrund seiner hohen Passgenauigkeit selbstplatzierend sein. Die Herstellung von Übertragungshilfen und die Herstellung eines Stereolithographiemodells entfallen. Auch die Herstellung einer Positiv-negativ-Form ist nicht erforderlich. Das erfindungsgemäße Verfahren kann die Nutzung eines medizinischen 3D-Datensatzes, eine 3D-Konstruktion eines virtuellen Modells und eine 3D-Umsetzung des virtuellen Modells in den erfindungsgemäßen Disktraktor vorsehen.

Die Erfindung wird nachstehend anhand von Ausführungsbeispielen, die die Erfindung nicht einschränken sollen, unter Bezugnahme auf die Zeichnungen näher erläutert. Dabei zeigen
- Fig. 1: eine Darstellung dreidimensionaler Ausdrucke der Unterkiefer von Patienten (Fig. 1a: Unterkiefer eines ersten Patienten; Fig. 1b: Unterkiefer eines zweiten Patienten);
- Fig. 2: eine schematische Vorderansicht des Unterkiefers eines Patienten; und
- Fig. 3: eine schematische Darstellung einer Ausführungsform eines erfindungsgemäßen Distraktors für die Verbreiterung des vorderen Unterkiefers eines Patienten (Fig. 3a: Draufsicht; Fig. 3b: Seitenansicht).

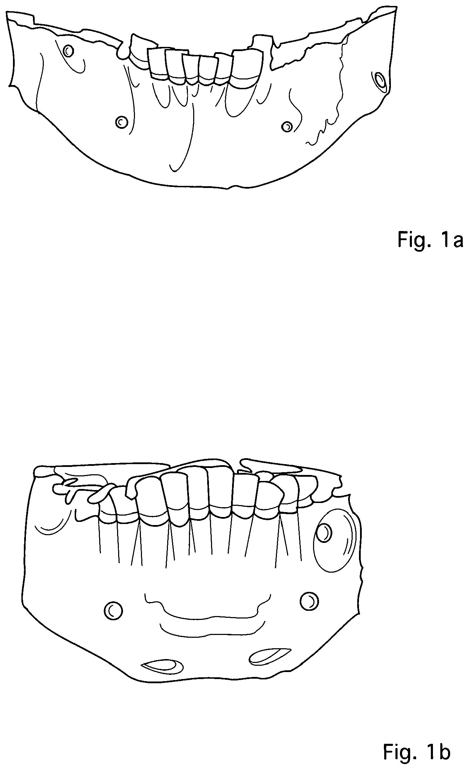

Fig. 1 zeigt dreidimensionale Ausdrucke der Unterkiefer zweier Patienten. Solche Ausdrucke können hergestellt werden, indem dreidimensionale Aufnahmen der Unterkiefer von Patienten mittels dreidimensionaler Röntgendiagnostik, beispielsweise dentaler Volumentomografie, angefertigt, anhand dieser Aufnahmen virtuelle Modelle der Unterkiefer erzeugt, und die virtuellen Modelle der Unterkiefer anschließend genutzt werden, um Modelle der Unterkiefer auszudrucken. Die Figuren 1a und 1b veranschaulichen die hohe interindividuelle anatomische Variabilität des Unterkiefers. Es sei darauf hingewiesen, dass die Fertigung dreidimensionaler Ausdrucke zur Ausführung des erfindungsgemäßen Verfahrens und zur Herstellung des erfindungsgemäßen Disktraktors nicht erforderlich ist. Es wird ein virtuelles Modell des Unterkiefers genutzt.

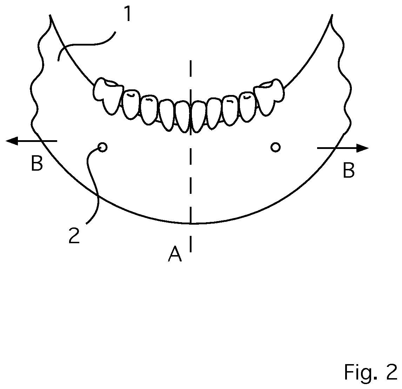

In Fig. 2 ist eine schematische Darstellung des Patienten-Vorderunterkiefers 1 gezeigt. In der Darstellung sind die Foramina mentale 2 gezeigt, die ein anatomisches Merkmal sind, dessen Lage von Patient zu Patient variiert. Die Foramina mentale 2 stellen somit eine der anatomischen Besonderheiten dar, die bei der Herstellung des erfindungsgemäßen Distraktors berücksichtigt werden müssen. Sie können daher als Referenzstrukturen diesen. Linie A in Fig. 2 kennzeichnet die Schnittführung bei einer vertikalen Osteotomie des Unterkiefers. Mittels des erfindungsgemäßen Distraktors sollen die an Linie A getrennten Teile des Unterkiefers in Richtung der Pfeile B voneinander wegbewegt werden.

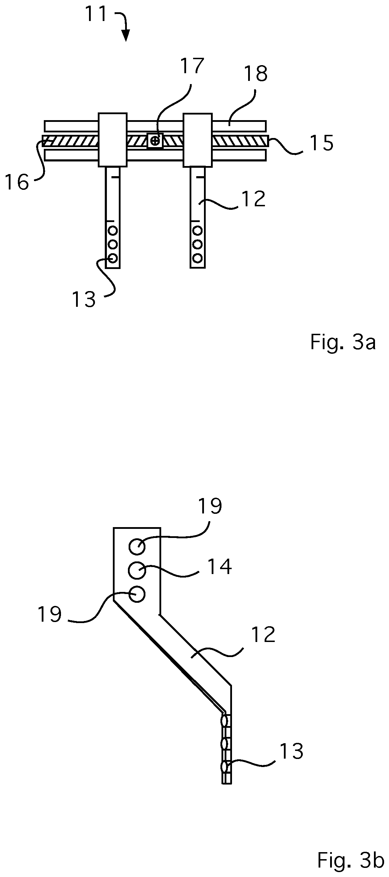

In den Figuren 3a und 3b ist eine Ausführungsform eines erfindungsgemäßen Distraktors 11 gezeigt. Es sei betont, dass das erfindungsgemäße Verfahren und der erfindungsgemäße Distraktor nicht auf diese Ausführungsform beschränkt sind, sondern auf jeden Distraktor gerichtet sind, der an die anatomischen Besonderheiten des Patienten angepasst ist und damit ein patientenspezifischer Distraktor ist. Die in den Figuren 3a und 3b gezeigte Ausführungsform des erfindungsgemäßen Distraktors 11 weist zwei Fixierungsarme 12 auf, die an dem Unterkiefer des Patienten befestigt werden sollen. In den Fixierungsarmen 12 sind erste Öffnungen 13 ausgebildet, durch die Schrauben geführt werden können, um die Fixierungsarme 12 an dem Unterkiefer 1 zu befestigen. Das erfindungsgemäße Verfahren erlaubt es, einen Distraktor 11 herzustellen, der an die spezifischen anatomischen Gegebenheiten des Unterkiefers eines Patienten angepasst ist. Die Lage, Anzahl und Orientierung der ersten Öffnungen kann damit so gewählt werden, dass die Verankerungsstellen, an denen Bohrungen im Kiefer 1 zur Aufnahme der Schrauben ausgebildet sind, eine optimale Lage haben, um das Behandlungsziel - die Verbreiterung des Vorderunterkiefers 1 - zu erreichen. Der in den Figuren 3a und 3b gezeigte Distraktor weist ferner zweite Öffnungen 14 in den Fixierungsarmen 12 auf, durch die ein Distraktionszylinder 15 geführt ist. Der Distraktionszylinder 15 weist Außengewinde 16 auf, die in Innengewinde, die in den zweiten Öffnungen 14 ausgebildet sind, eingreifen können. Mittels eines Stellelementes 18 kann der Distraktionszylinder 15 gedreht werden, wodurch der Abstand zwischen den beiden Fixierungsarmen 12 verändert werden kann. Ferner sind Führungsstifte 18 vorgehen, die durch dritte Öffnungen 19 in den Fixierungsarmen 12 geführt sind.

Es ist in den Figuren 3a und 3b in Verbindung mit Fig. 1 zu erkennen, dass die Länge und Breite der Fixierungsarme 11 und die Lage der ersten und zweiten Öffnungen 13, 14 an die anatomischen Besonderheiten des Unterkiefers 1 des zu behandelnden Patienten angepasst werden sollten, um ein optimales Behandlungsergebnis zu erreichen, das Anbringen des Distraktors 11 am Unterkiefer 1 zu erleichtern, die dazu erforderliche Zeit zu verkürzen und die Beeinträchtigung des Patienten, beispielsweise durch Setzen der Schrauben im Bereich der Nervenkanäle zu verhindern. Das erfindungsgemäße Verfahren sieht somit vor, zunächst eine dreidimensionale Aufnahme des Unterkiefers 1 des Patienten, vorzugsweise mittels dreidimensionaler Röntgendiagnostik, anzufertigen, auf Basis der dreidimensionalen Aufnahme ein virtuelles Modell eines Distraktors 11 anzufertigen, dass an die anatomischen Besonderheiten des Unterkiefers 1 angepasst ist, und schließlich das virtuelle Modell des Distraktors 11 in einen realen Distraktor 11 zu überführen, der vorzugsweise mittels 3D-Titandruck hergestellt werden kann. Der so erhaltene Distraktor 11 ist an die individuellen Gegebenheiten des Patienten angepasst und damit vollständig individualisiert.

Es kann vorgesehen sein, dass nur die beiden Fixierungsarme 12 und die Schrauben zur Befestigung der Fixierungsarme 12, also der skelettale Anteil des Distraktors 11, mittels 3D-Titandruck anhand des virtuellen Modells hergestellt werden, während für den Distraktionszylinder 15 und die Führungsstifte, also den nicht-skelettalen Anteil des Distraktors 11, Standardelemente verwendet werden, die aus Titan vorgefertigt wurden. Auch auf diese Weise wird ein vollständig individualisierter, d. h. an die anatomischen Besonderheiten des Patienten angepasster Destraktor erhalten.

### Bezugszeichenliste

- 1: Unterkiefer
- 2: Foramen mentale

- 11: Distrakor
- 12: Fixierungsarm
- 13: erste Öffnung
- 14: zweite Öffnungen
- 15: Distraktionszylinder
- 16: Gewinde
- 17: Stellelement
- 18: Führungsstift
- 19: dritte Öffnung

## Patentansprüche

1. Verfahren zur Herstellung eines Distraktors (11) zur skelettalen Befestigung an einem Unterkiefer (1) eines Patienten, wobei der Distraktor (11) exakt an die anatomischen Gegebenheiten des Patienten angepasst ist, umfassend die Schritte
(a) Anfertigen einer dreidimensionalen Aufnahme des Unterkiefers (1) des Patienten oder von Teilen des Unterkiefers (1);
(b) Anfertigen eines virtuellen Modelles eines patientenspezifischen Distraktors (11) unter Verwendung der dreidimensionalen Aufnahme; und
(c) Herstellung des Distraktors (11) oder Teilen davon anhand des virtuellen Modelles mittels 3D-Druckens.

2. Verfahren nach Anspruch 1, **dadurch gekennzeichnet, dass** die dreidimensionale Aufnahme mittels eines Röntgenverfahrens angefertigt wird.

3. Verfahren nach einem der vorstehenden Ansprüche, **dadurch gekennzeichnet, dass** die dreidimensionale Aufnahme mittels Computertomographie, Magnetresonanztomographie, dentaler Volumentomographie oder anderer 3D-Aufnahmetechniken angefertigt wird.

4. Verfahren nach einem der vorstehenden Ansprüche, **dadurch gekennzeichnet, dass** die dreidimensionale Aufnahme als medizinischer Datensatz bereitgestellt wird.

5. Verfahren nach einem der vorstehenden Ansprüche, **dadurch gekennzeichnet, dass** ein virtuelles Modell des Unterkiefers (1) des Patienten oder eines Teiles des Unterkiefer (1) angefertigt wird.

6. Verfahren nach einem der vorstehenden Ansprüche, **dadurch gekennzeichnet, dass** Referenzstrukturen des Unterkiefers (1) oder eines Teils davon anhand der dreidimensionale Aufnahme bestimmt werden und die Referenzstrukturen zur Anfertigung des virtuellen Modells des patientenspezifischen Distraktors (11) verwendet werden.

7. Verfahren nach Anspruch 6, **dadurch gekennzeichnet, dass** die Referenzstrukturen aus der Gruppe ausgewählt sind, die den Foramen mentale (2) und Nervenkanäle umfasst.

8. Verfahren nach einem der vorstehenden Ansprüche, **dadurch gekennzeichnet, dass** Verankerungsstellen zum Befestigen des Distraktors (11) am Unterkiefer (1) des Patienten anhand der dreidimensionalen Aufnahme bestimmt werden.

9. Verfahren nach einem der vorstehenden Ansprüche, **dadurch gekennzeichnet, dass** zumindest die Teile des Distraktors (11), die in unmittelbaren Kontakt mit dem Unterkiefer gebracht werden sollen, anhand des virtuellen Modells hergestellt werden.

10. Verfahren nach einem der vorstehenden Ansprüche, **dadurch gekennzeichnet, dass** der Distraktor (11) Fixierungsarme (12) zur Befestigung an dem Unterkiefer (1) des Patienten, Verbindungselemente zum Befestigen der Fixierungsarme (12) am Kiefer (1) des Patienten sowie einen Distraktionszylinder (15) zum Verändern des Abstandes zwischen den Fixierungsarmen (12) aufweist, wobei nur die Fixierungsarme (12) oder nur die Fixierungsarme (12) und die Verbindungselemente anhand des virtuellen Modells hergestellt werden.

11. Verfahren nach einem der vorstehenden Ansprüche, **dadurch gekennzeichnet, dass** der Distraktor (11) oder Teile davon anhand des virtuellen Modells mittels 3D-Titandrucken hergestellt werden.

12. Distraktor zur skelettalen Befestigung an einem Unterkiefer eines Patienten, wobei der Distraktor (11) mittels des Verfahrens gemäß einem der Ansprüche 1 bis 11 hergestellt wurde, so dass er an den Unterkiefer (1) des Patienten exakt angepasst ist.

13. Distraktor nach Anspruch 12, **dadurch gekennzeichnet, dass** der Distraktor (11) Fixierungsarme (12) zur Befestigung an dem Unterkiefer (1) des Patienten, Verbindungselemente zum Befestigen der Fixierungsarme (12) am Unterkiefer (1) des Patienten sowie einen Distraktionszylindern (15) zum Verändern des Abstandes zwischen den Fixierungsarmen (12) aufweist, wobei nur die Fixierungsarme (12) oder nur die Fixierungsarme (12) und die Verbindungselemente anhand des virtuellen Modells hergestellt werden.

14. Distraktor nach Anspruch 12, **dadurch gekennzeichnet, dass** der Distraktor (11) einen skelettalen Anteil und einen nicht-skelettalen Anteil aufweist, wobei der skelettale Anteil anhand des virtuellen Modells hergestellt ist und der nicht-skelettale Anteil aus vorgefertigten Elementen besteht.

## Claims

1. A method for manufacturing a distractor (11) for skeletal attachment to a jaw (1) of a patient, wherein the distractor (11) is exactly adapted to the anatomic conditions of the patient, comprising the steps of
(a) generating a three-dimensional image of the jaw (1) of the patient or of parts of the jaw (1);
(b) generating a virtual model of a patient-specific distractor (11) using the three-dimensional image; and
(c) manufacturing the distractor (11) or parts thereof with the help of the virtual model by means of 3D printing.

2. The method according to claim 1, **characterized in that** the three-dimensional image is generated by means of an X-ray method.

3. The method according to any of the preceding claims, **characterized in that** the three-dimensional image is generated by means of computer tomography, magnetic resonance tomography, dental volume tomography, or other 3D image-generating techniques.

4. The method according to any of the preceding claims, **characterized in that** the three-dimensional image is provided as a medical data record.

5. The method according to any of the preceding claims, **characterized in that** a virtual model of the jaw (1) of the patient or a part of the jaw (1) is generated.

6. The method according to any of the preceding claims, **characterized in that** reference structures of the jaw (1) or a part thereof are determined with the help of the three-dimensional image and the reference structures are used to generate the virtual model of the patient-specific distractor (11).

7. The method according to claim 6, **characterized in that** the reference structures are selected from the group comprising the mental foramen (2) and nerve canals.

8. The method according to any of the preceding claims, **characterized in that** anchorages for attaching the distractor (11) to the jaw (1) of the patient are determined with the help of the three-dimensional image.

9. The method according to any of the preceding claims, **characterized in that** at least parts of the distractor (11) that are to be directly contacted with the jaw are manufactured with the help of the virtual model.

10. The method according to any of the preceding claims, **characterized in that** the distractor (11) has fixing arms (12) for attachment to the jaw (1) of the patient, connecting elements for attaching the fixing arms (12) to the jaw (1) of the patient, as well as a distraction cylinder (15) for changing the distance between the fixing arms (12), wherein only the fixing arms (12) or only the fixing arms (12) and the connecting elements are manufactured with the help of the virtual model.

11. The method according to any of the preceding claims, **characterized in that** the distractor (11) or parts thereof are manufactured with the help of the virtual model by means of 3D titanium printing.

12. A distractor for skeletal attachment to a jaw of a patient, wherein the distractor (11) was manufactured by means of the method according to any one of claims 1 to 11, so that it is exactly adapted to the jaw (1) of the patient.

13. The distractor according to claim 12, **characterized in that** the distractor (11) has fixing arms (12) for attachment to the jaw (1) of the patient, connecting elements for attaching the fixing arms (12) to the jaw (1) of the patient, as well as a distraction cylinder (15) for changing the distance between the fixing arms (12), wherein only the fixing arms (12) or only the fixing arms (12) and the connecting elements are manufactured with the help of the virtual model.

14. The distractor according to claim 12, **characterized in that** the distractor (11) has a skeletal portion and a non-skeletal portion, wherein the skeletal portion is prepared with the help of the virtual model and the non-skeletal portion consists of prefabricated elements.

## Revendications

1. Procédé pour la fabrication d'un distracteur (11) pour la fixation osseuse sur une mandibule (1) d'un patient, le distracteur (11) étant précisément ajusté aux conditions anatomiques du patient, comprenant les étapes
(a) réalisation d'une image tridimensionnelle de la mandibule (1) du patient ou de parties de la mandibule (1);
(b) réalisation d'un modèle virtuelle d'un distracteur spécifique au patient (11) sur l'utilisation d'une image tridimensionnelle; et
(c) fabrication du distracteur (11) ou de pièces de ce dernier sur la base du modèle virtuel à l'aide de l'impression 3D.

2. Procédé selon la revendication 1, **caractérisé en ce que** l'image tridimensionnelle est réalisée au moyen d'un procédé radiographique.

3. Procédé selon l'une des revendications ci-dessus, **caractérisé en ce que** l'image tridimensionnelle est produite au moyen d'une tomographie assistée par ordinateur, d'une tomographie par résonance magnétique, d'une tomographie de volume dentaire ou d'autres techniques d'imagerie 3D.

4. Procédé selon l'une des revendications ci-dessus, **caractérisé en ce que** l'image tridimensionnelle est fournie sous la forme d'un enregistrement de données médicales.

5. Procédé selon l'une des revendications ci-dessus, **caractérisé en ce qu'**un modèle virtuel de la mandibule (1) du patient ou d'une partie de la mandibule (1) est réalisé.

6. Procédé selon l'une des revendications ci-dessus, **caractérisé en ce que** des structures de référence de la mandibule (1) ou d'une partie de celle-ci sont déterminées à partir de l'image tridimensionnelle et les structures de référence sont utilisées pour produire le modèle virtuel du distracteur spécifique au patient (11).

7. Procédé selon la revendication 6, **caractérisé en ce que** les structures de référence sont choisies à partir du groupe comprenant le foramen mental (2) et des canaux nerveux.

8. Procédé selon l'une des revendications ci-dessus, **caractérisé en ce que** des points d'ancrage pour la fixation du distracteur (11) sur la mandibule (1) du patient sont déterminés sur la base de l'image tridimensionnelle.

9. Procédé selon l'une des revendications ci-dessus, **caractérisé en ce qu'**au moins des parties du distracteur (11) qui doivent être appliquées en contact direct avec la mandibule sont fabriqués à partir du modèle virtuel.

10. Procédé selon l'une des revendications ci-dessus, **caractérisé en ce que** le distracteur (11) comprend des bras de fixation (12) pour la fixation sur la mandibule (1) du patient, des éléments de liaison pour fixer les bras de fixation (12) sur la mâchoire (1) du patient et un vérin de distraction (15) pour la modification de la distance entre les bras de fixation (12), seuls les bras de fixation (12) ou seuls les bras de fixation (12) et les éléments de liaison étant fabriqués à partir du modèle virtuel.

11. Procédé selon l'une des revendications ci-dessus, **caractérisé en ce que** le distracteur (11) ou des parties de celui-ci sont fabriqués à partir du modèle virtuel au moyen d'une impression 3D en titane.

12. Distracteur pour fixation osseuse sur la mandibule d'un patient, le distracteur (11) ayant été fabriqué au moyen du procédé selon l'une quelconque des revendications 1 à 11 de manière à s'adapter exactement à la mandibule (1) du patient.

13. Distracteur selon la revendication 12, **caractérisé en ce que** le distracteur (11) comprend des bras de fixation (12) pour la fixation sur la mandibule (1) du patient, des éléments de liaison pour fixer les bras de fixation (12) sur la mandibule (1) du patient ainsi qu'un vérin de distraction (15) pour la modification de la distance entre les bras de fixation (12), seuls les bras de fixation (12) ou seuls les bras de fixation (12) et les éléments de liaison étant fabriqués à partir du modèle virtuel.

14. Distracteur selon la revendication 12, **caractérisé en ce que** le distracteur (11) comprend une partie osseuse et une partie non osseuse, la partie osseuse étant produite à partir du modèle virtuel et la partie non osseuse étant constituée d'éléments préfabriqués.
